# EUROPEAN PATENT SPECIFICATION

(11) **EP 4 171 727 B1**
(45) Date of publication and mention of the grant of the patent: **30.07.2025**
(21) Application number: 21733984.5
(22) Date of filing: 18.06.2021
(51) Int. Cl.: A61N 1/372, A61B 5/00, A61N 1/375

(54) **MEDICAL SYSTEM COMPRISING AN ACTIVE IMPLANTABLE MEDICAL DEVICE AND METHOD FOR OPERATION OF THE SAME**
MEDIZINISCHES SYSTEM MIT EINER AKTIVEN IMPLANTIERBAREN MEDIZINISCHEN VORRICHTUNG UND VERFAHREN ZU DESSEN BETRIEB
SYSTÈME MÉDICAL COMPRENANT UN DISPOSITIF MÉDICAL IMPLANTABLE ACTIF ET SON PROCÉDÉ DE FONCTIONNEMENT

(30) Priority: 30.06.2020 US 202063045874 P; 28.07.2020 EP 20188145
(43) Date of publication of application: 03.05.2023
(73) Proprietor: BIOTRONIK SE & Co. KG, 12359 Berlin (DE)
(72) Inventor: TAFF, Brian M., Portland, OR 97217 (US)
(74) Representative: Biotronik Corporate Services SE
(86) International application number: PCT/EP2021/066584
(87) International publication number: WO 2022/002636

(56) References cited:
- WO-A1-2007/027570
- WO-A1-2008/118908
- GB-A- 2 569 157
- US-A1- 2014 222 109
- US-A1- 2019 201 701

## Description

The present invention refers to a medical system comprising an implantable medical device and an extracorporeal device as well as a method for operation of the same.

Active implantable medical devices (IMD), for example implantable stimulation devices such as pacing devices (e.g. implantable cardiac pacemakers or implantable leadless pacemakers (ILPs)) are well known medical devices that allow stimulation of the heart of a patient. In general, such medical devices are battery operated and a stimulation component is directly implanted into the heart's ventricle or atrium. Standard implantable cardiac pacemakers provide at least one elongated stimulation lead that reaches from the device housing into a heart chamber where the tip of said lead is anchored. ILPs are miniaturized pacing devices that are entirely implanted into the heart chamber. Implantable pacemakers or ILPs are used for patients who suffer from bradycardia, (i.e., a condition wherein the heart beats too slowly to fulfill the patient's physiologic needs). The implantable pacemaker or ILP applies electrical stimulation to the heart to generate a physiologically appropriate heart rate. Other types of active implantable medical devices that are deeply incorporated in the patient's body are, for example, leadless pulmonary pressure sensors, or even, stents or valves that (in targeted, future embodiments are anticipated to) incorporate active on-board electronics.

An IMD such as an ILP presents a highly constrained power budget in balancing competing needs for both long therapeutic service and compact sizing. In addition, their residence within the heart at distances well below the surface of the patient's skin present fundamental roadblocks to the capacity to viably relay RF signaling from the IMD to bedside monitoring devices commonly used to pass information onward to remote monitoring infrastructures.

Prior to the early 2000s, active IMDs had not advanced to an extent capable of incorporating RF-enabled telemetry for the reporting of information on IMD/patient interactions between follow-up. The two primary means for notification within this context centered on auditory signaling from the device (e.g., a "beep") or the instatement of changes in the therapy output that could be "felt" by the patient. Given the limited nature of these notifications, they tended to be administered only as cues that the IMD had entered the prolonged service period (i.e., passing the early replacement indicator condition).

It is not clear how such methods could readily scale to offer user-friendly reporting on the true variety of patient/IMD conditions that would offer clinical benefit. While the symptomatic therapy output strategy has been preserved in many modem devices, it remains limited to applications where the user is notified of an impending need for device replacement. Auditory notifications have been recognized as an undesired format and have been largely (if not entirely) retired as a clumsy means for notification with benefits that struggle to outweigh their tendency to instill panic in patients and their loved ones.

For modern, larger IMDs (excluding leadless pacemakers, but inclusive of neuro devices, and leaded IPGs and ICDs), access to larger in-implant battery support and their anatomical residence in shallow subcutaneous pockets means that a larger power budget facilitates the passage of data across a relay channel with manageable attenuation characteristics using now readily available RF telemetry capabilities. As such, these common IMDs are able to send critical patient/IMD information to external resources without compromising device longevity in significant ways and furthermore by leveraging a patient compliance characteristic that is limited only to the patient being "within range" of a remote (typically bedside) device at times when data relay to remote services infrastructure occur (typically in the middle of the night).

For smaller IMDs such as leadless pacemakers, an alternative patient compliance model has been promoted/suggested to offer, at least remedial, remote monitoring support. In this approach, it becomes the patient's duty to place a wand-based external device over their implant on a prescribed basis to extract data from the deep, low-power IMD and pass it onward to the remote monitoring services infrastructure. In addition, any coordinated, co-prescribed medication regimens are managed by requiring that the patient remembers to self-administer their drugs.

The known RF-enabled solutions for data extraction from the IMD only prove viable for subcutaneously implanted devices with large batteries as
1.) a substantial amount of energy is needed to generate the RF output, and
2.) said output needs to propagate from the IMD through a medium that is not grotesquely capable of attenuating its receipt by an external device. Given that smaller IMDs such as ILPs are neither capable of incorporating large battery supplies nor positioned in environments where the surrounding physiology avoids direct competition with RF data telemetry, legacy product approaches to reporting patient status and enabling remote monitoring support do not prove directly transferrable.

As for the patient compliance model promoted for smaller IMDs such as ILPs, it places new responsibilities on the patient that have not been commonplace to remote monitoring as it has been known and promoted within the cardiac rhythm management (CRM) industry. As such, patients are much more inclined to forget that they need to collect the data which typically leads to irregular collection. Spotty data is sometimes better than none at all, but not always. In addition, some patient's may tend to "overdo it" and falsely imagine that the more they interrogate their implant, the better they will be able to manage their personal care. Unfortunately, these anxiety-motivated check-ins can tax the implant's power resources and shorten device service times if done excessively regularly and without built-in measures to throttle the impact of their frequency.

Other medical systems are known from WO 2007/027570 A1 and WO 2008/118908 A1.

Accordingly, there is a need for a medical system that enables remote monitoring for deep active IMDs that does not require the patient to remember to periodically check in on patient/IMD conditions. Further, it is desirable to minimize the demand on the deep IMD's battery any time that messaging is relayed to an external device and lean on other more power-enabled systems to aid in relaying data from the deep IMD.

The above problem is solved by the medical system defined in claim 1 and the method defined in claim 10.

The invention is defined by the appended claims.

In the present disclosure, a medical system is described comprising an active implantable medical device (IMD) and an extracorporeal device attached to a patient's skin, wherein the IMD comprises
- a measurement unit adapted to determining data regarding at least one bodily parameter of the patient when the IMD is implanted within a body of the patient,
- a processor adapted to process data regarding the at least one bodily parameter and to detect at least one condition associated with a plurality of predefined conditions based on the data,
- a communication unit adapted to periodically send (e.g., 1x/30min for upwards of 24 hours) an intra-body communication signal representing the at least one detected condition to the extracorporeal device,

wherein the extracorporeal device comprises
   - a transceiver unit adapted to receive the intra-body communication signal,
   - a notification unit adapted to provide at least one predefined notification signal to the patient in order to motivate the patient to perform a predefined action based on the received intra-body communication signal,
wherein the transceiver unit of the extracorporeal device is further adapted to send a termination signal to the communication unit of the IMD, and
the communication unit of the IMD is further adapted to receive the termination signal and to subsequently terminate sending the intra-body communication signal which was initiated by the previously at least one detected condition.

The transceiver unit of the extracorporeal device is configured to send the termination signal automatically to the communication unit of the IMD when the transceiver unit of the extracorporeal device receives the intra-body communication signal. Additionally or alternatively, the transceiver unit of the extracorporeal device is configured to send the termination signal to the communication unit of the IMD when the notification signal is acknowledged by the patient.

Such an acknowledgement by the patient creates additional security that the notification signal has been consciously perceived by the patient. This improves the effectiveness of the notification signal in the context of communication.

Moreover, in cases where interrogation by means of the patient device is requested by the IMD, beaconing from the IMD is silenced as soon as the patient device interrogates the IMD, thereby resetting the IMD for subsequent data collection. According to a further example, the IMD beaconing is stoppable by at least one further mechanism. For example, the extracorporeal device sends a termination signal to the IMD upon receiving the beaconing. That example saves battery life of the IMD in case an interrogation cannot be performed for a longer time period.

According to an example, termination of the beaconing is linked to a time-out process, where beaconing stops after a certain number of transmission attempts or a certain time interval expired. The time-out process serves maximized IMD longevity.

Preferably, the user can configure what conditions to generate beacons and change those settings according to his/her need. For example, if the user is aware that the patient has atrial fibrillation (Afib) and it is an unmanageable condition, one may not want to waste IMD longevity by having the implant constantly sending over beacons to report Afib.

Moreover, the notifications on the extracorporeal device could be cleared by the user through an acknowledgement process. Some such clearing may be, in cases where the system recognizes that a requested IMD interrogation had been performed by the patient device. In such a case, the successful transmission of the patient data to the remote services infrastructure could be used to let the patient device inform the IMD of its success, which, in turn, could subsequently be transmitted to the worn extracorporeal device.

According to some examples, the remote services infrastructure sends information to the extracorporeal device via the patient device to inform the patient. As an example, that function can be used by a care provider for remote follow up notifications. For instance, a 2 week regimen of daily, remote follow-up data collection can be notified or an adherence to a co-prescribed pill-taking regimen. In such instances, the patient device which is directly linked to the remote services infrastructure, would receive either a series of daily messages, or a calendar block with a set of embedded daily alarms. In either case, the patient device would, via Bluetooth, wireless RF telemetry or otherwise pass the information to the worn extracorporeal device. Such support would likely best occur by having the patient device residing next to the patient's bed. At night, while the patient is near the patient device, it could then pass the daily calls or schedule block to the worn extracorporeal device. The content that was received by the extracorporeal device would then present notifications on the worn device over a series of days or in association with a specifically timed inquiry from the clinical care team. The patient would see these notifications on their worn extracorporeal device and then be compelled to take specific actions. Such actions could include daily interrogations and/or requests that patients provide feedback on their compliance with co-prescribed care support medication - blood thinner, for example.

According to an example, the notifications on the extracorporeal device can take a variety of forms and each can be distinct based on their origin/type. The separate patient device can also provide notifications of its own, predominantly centered on reporting the success/failure/pending/etc. status of efforts to transmit IMD interrogation data to the remote services environment.

The inventive method and the inventive system, first and foremost facilitates a means to revise the remote monitoring approach to relay information only when doing so adds value to the patient care model through condition-based/triggered cues for data relay. Because a bulk of traditional daily monitoring data from IMDs represents output that lacks meaningful clinical implication/consequence, the strategy centers data transmission on emergent IMD-recognized patient/device interactions and/or prescribed interactions from the patient's clinician administered remotely.

One such embodiment, specific to CRM support, could, among other considerations, offer the capacity for the device itself to trigger the reporting of high pacing capture thresholds (PCTs) and/or atrial fibrillation (Afib, especially "silent" varieties. Such IMD-triggered cues could additionally include the ability to store coordinated in-device IEGM snapshots and other relevant condition-specific statistics. Beyond sidestepping the power consumption that would be needed by the system when compared to legacy approaches that relay IMD/patient data on a daily basis, the approach avoids inundating the patient's care support team with large volumes of information that might need to be mined for the discovery of truly important findings.

In varied examples, the remote services infrastructure could furthermore push care management regimens to the patient's extracorporeal device either directly in embodiments where the extracorporeal device is itself connected to the remote services center or, in more likely embodiments, by means of a separate patient device that is directly connected to the remote service center and can wirelessly relay such information to the worn extracorporeal device. These care management regimens would either embody a series of individual messages delivered from the remote services infrastructure on a prescribed schedule (e.g. daily for 2 weeks) or the installation of an entire schedule/calendar (complete with an embedded series of prescribed notification alarms) within the local storage memory of the worn extracorporeal device. Whether pushed from the remote services infrastructure as individual messages or as a block regimen of notification alarms, the method and system would enable calls for patient engagement over a specific time course.

In these follow-up focused examples, the pushed requests/schedules from the remote services infrastructure would encourage the user to interrogate the deep implant at specific times using the separate wand-enabled patient device. Further, such remote services infrastructure pushes would be readily able to support messaging encouraging compliance with co-prescribed drug regimens. This latter type of notification (again, whose notifications would be made available to the user by means of the worn extracorporeal device), would not necessarily be tied to requests that the patient perform subsequent interactions with the wand-enabled patient device, but they could still incorporate a feedback mechanism where patient interactions with the extracorporeal device could inform remote monitoring services (again, in likely examples relaying such messaging through the patient device) as to whether medications had been taken as directed or otherwise.

For patient/IMD condition-triggered use of this system, the mechanics would involve having the communication unit of the IMD (likely in coordination with the in-device processor) report detected conditions to the outside world initially through an intra-body communication channel - said mechanism including accommodation to encode condition type and/or other minimal identifiers within the relayed content. Such intra-body communication would likely occur by means of acoustic, electrical, and/or an impedance-based physical layer implementation. The communication unit of the IMD (again, in likely coordination with the in-device processor) would additionally support further data relay to the outside world through subsequent inductive interactions between the deep implant and a separate external coil-enabled patient device - the latter being connected to a remote services infrastructure and independent of the worn extracorporeal device. The patient device could locally store the collected IMD data and transmit it onward to the remote services infrastructure. In coordination with this relay (perhaps subject to confirmed receipt by the remote services infrastructure) the external wand-based device could potentially also coordinate the halting of implant beacons via direct inductive communication with the IMD. The wand-based patient device may, itself, furthermore operate to notify the patient that interrogated information has successfully been collected and/or relayed to the remote service center. Such support could be provided by a number of means which would include (but are not limited to) LED-based indicator lighting, LCD-based visual iconography, or auditory signaling (e.g., success "chime" sounds). Further, the method may comprise means for the patient device to initiate the clearing of notifications on the separate extracorporeal device to effectively "reset" the system and await any follow-on detected conditions (likely by having the patient device communicate with the IMD and then having the IMD communicate via intra-body communication with the worn extracorporeal device).

Whether the remote monitoring support feature is driven by IMD-recognized conditions or scheduling pushed from the remote services infrastructure, the method and system outlined in the present disclosure serves to offset some of the burdens associated with either
1.) a complete lack of any remote monitoring capabilities or
2.) reliance on a system that depends upon patients following a routine that, at best, might be encouraged via periodic texts from the clinical support facility (or similar notification schemes, wholly unconnected from the deep implant, any information the IMD might be collecting with regard to patient/device interactions, and/or a prescription of care from the provider where compliance could make a meaningful difference in outcomes).

Moreover, the present disclosure also relates to a method for operation of a medical system comprising an active implantable medical device (IMD), such as an ILP, and an extracorporeal device attached to a patient's skin is provided comprising the following steps:
- determining data regarding at least one bodily parameter of the patient by a measurement unit of the IMD when the IMD is implanted within a body of the patient,
- processing the data regarding the at least one bodily parameter and detecting at least one condition of a plurality of predefined conditions based on the data by a processor of the IMD,
- periodically sending an intra-body communication signal representing the at least one detected condition by a communication unit of the IMD to the extracorporeal device,
- receiving the intra-body communication signal by a transceiver unit of the extracorporeal device,
- providing at least one predefined notification signal to the patient to motivate the patient to perform a predefined action based on the received intra-body communication signal,
- sending a termination signal by the transceiver unit of the extracorporeal device to the communication unit of the IMD, and
- receiving the termination signal by the communication unit of the IMD and subsequently terminating sending the intra-body communication signal which was initiated by the previously at least one detected condition.

According to an example, the intra-body communication signal is sent once in 30 minutes, 1 hour, 2 hours... up to 24 hours.

Preferably, once the intra-body communication signal is received by the transceiver unit of the extracorporeal device, the extracorporeal device provides at least one predefined notification signal to the patient. Once the extracorporeal device was able to notify the patient, the termination signal is sent automatically from the transceiver unit of the extracorporeal device to the communication unit of the IMD in attempt to terminate the further transmissions of the intra-body communication signal by the communication unit of the IMD. By sending the termination signal to the IMD directly upon notification of the patient, the IMD is prevented from further transmitting redundant intra-body communication signals, which saves IMD battery lifetime.

According to another example, the notification signal is acknowledged by the patient before the termination signal is sent by the transceiver unit of the extracorporeal device to the communication unit of the IMD.

Alternatively, a patient device which is in communication with the IMD may send the signal for triggering a termination signal to the extracorporeal device. Moreover, a remote services infrastructure which communicates with the patient device said signal, which is then forwarded by the patient device to the extracorporeal device.

According to an example, the system and approach would also have means signaling the IMD that it had been made aware of the condition it had recognized using a termination signal. This termination signal would for example come from the extracorporeal device simply receiving the IMD intra-body communication signal (or also called "beacon" herein) and then the extracorporeal device would tell the IMD to stop transmitting its beacons. The IMD could then seek new conditions as if it had been "reset" for added monitoring.

According to the present disclosure, an active IMD is an implantable medical device which provides an active therapeutic impact to a patient's body, for example by providing electrical or magnetic stimulation to an organ or vessel; by delivering a medicament to the patient's body; or by providing an active mechanical movement to the body or parts of it. In one example, the present disclosure is directed to deep implanted, small IMDs, for example ILPs, which do not provide large space for an energy source such as a battery and/or are not reliably accessible by radio-frequency (RF) signaling for information transmission.

The noted bodily parameter may be any electrical signal of the body, for example from a heart's region such as an acquired impedance value, or sense input from the electrocardiogram, any material concentration of the body, for example the concentration of any material component contained in the patient's blood, or any electromagnetic signature of the body, for example any nuclear magnetic resonance signal. This bodily parameter of the patient is detected by the measurement unit of the IMD, for example continuously.

The above method involves embedding an algorithmic capability within the deepimplanted IMD to enable condition detection from the data of the above bodily parameter, wherein condition detection means that a prescribed set of conditions have been observed in one or a sequence of analyzed measurements of the bodily parameter. For CRM devices, such algorithmic capabilities may include (but are not limited to): determinations of the presence of atrial fibrillation (AF), especially "silent" varieties, or high pacing capture thresholds (PCTs). The processor compares the recently determined data of the at least one bodily parameter with at least one predefined threshold, template data structure representative of the condition sought, or other screening strategy to decide whether the at least one predefined condition (e.g. an illness or pathologic situation) has occurred.

The noted extracorporeal device is a device comprising a processor and a transceiver unit electrically connected to the processor, wherein the transceiver unit may be integrated into the processor, for receiving the intra-body communication signal and for sending the termination signal. The extracorporeal device may comprise a display, a loudspeaker, a microphone and/or a tactile noticeable unit, each of which may provide the at least one predefined notification signal, for example a visual, an acoustic and/or tactile noticeable signal. The acoustic signal may be in the ultrasound region. For example, the display may provide a text message, a symbol or a blinking signal to the patient. The extracorporeal device is, for example, a smart watch, a wrist band, a mobile phone worn in direct physical contact with the patient's skin and/or a patch device.

In case at least one predefined condition is detected, the communication unit of the IMD starts sending an intra-body communication signal in periodic fashion. This means that upon detection of at least one such condition, the IMD may transmit a short encoded beacon via intra-body communication (i.e. within the patient's body) directly to the transceiver unit of the extracorporeal device attached to the surface (i.e. skin) of the patient. The short beacon is primarily constructed to make it clear that it represents a deliberate signal/output from the implant but would also include enough remedial information to indicate the triggering condition type.

Encoding could take the form of different frequency settings in "ping" outputs from the IMD where the frequency content of the "pings" could report the condition recognized. Other formats could simply provide short bit sequences where the specific relayed ordering of ones and zeros is affiliated with different recognized conditions. The term "encoded" in this scenario means that IMD and the wearable device speak a common language. As an example, if a single byte is sent as the message, for example 11011000, the receiver would need to have an appropriate lookup table, logic, or software to interpret this message as meaning "low pacing capture thresholds" (or whatever relevant, agreed upon definitions were used to reflect the found condition(s)). Encoding is simply formatting the data in ways that the receiver and transmitter have agreed upon for passing messaging. Another of encoding is frequency encoding for example. Instead of sending the byte mentioned above, a single "ping" at 32 kHz could mean the same thing, while a "ping" at 100 kHz could mean "high rate condition".

The term 'short' in 'short encoded beacon' means that it the signal should not demand that the implant transmitter remains in an ON state for a duration that is sufficient enough to meaningfully reduce the product service time. Typically, one or a few 8-bit bytes of information would likely prove sufficient.

A single "ping" can be used as a short encoded beacon, wherein the frequency of the "ping" is distinctly associated with different patient conditions.

Moreover, the intra-body communication beacon may be transmitted through the patient's body using an acoustic signal, an electrical signal and/or an impedance-based signal. Any such beaconing from the IMD travels through the patient's body to the transceiver unit of the extracorporeal device. In examples where impedance-based signaling is leveraged it may employ an applied oscillating electrical field emergent from the extracorporeal device and an ON/OFF switching response from the IMD that ultimately changes the impedance of the conductive medium surrounding the IMD and is subsequently detectable by the extracorporeal device as described further in US 2014/0222109 A1.

The beacon may, perhaps most reasonably, be transmitted from the IMD in a periodic fashion (e.g. repeatedly, for example once per hour or every 1/2 hour or every minute) for a set duration of time (e.g. upwards of 24-hours) with a capacity for its transmission to be halted prior to timeout (e.g. prior to the end of a 24-hour duration) in response to appropriate signaling received from the extracorporeal device. This halting command input, or termination signal from the extracorporeal device would be expected to be transmitted from the extracorporeal device, again, via above described intra-body communication.

The receipt of the beacon from the deep IMD by the transceiver unit of the extracorporeal device would trigger this device to display a predefined notification signal, for example an icon, a legible message, generate a tactile/haptic response (e.g. vibrate), make a sound (e.g. chime/or beep), or trigger an affiliated other, e.g. Bluetooth-connected, data processing device, e.g. a mobile device, or otherwise to produce similar calls for attention in order to motivate the patient (i.e. to instruct the patient), to perform a predefined action. The predefined notification signal depends on the sent intra-body communication signal received from the IMD because for different IMD-recognized conditions a different notification signal may be used to motivate the patient to perform different actions.

The notification signal comprises one or more of the following:
- a reporting to the user of the condition observed by the IMD, wherein the reporting may explicitly name the observed condition,
- an invitation for the patient to establish a data communication connection to the IMD using a patient device,
- an invitation for the patient to take a medication and/or
- an invitation for the patient to visit a healthcare professional.

According to an example, the invitation for the patient to establish a data communication connection to the IMD using a patient device comprises a request to place the patient device in proximity of the IMD (e.g. of the patient's chest).

Preferably, after successfully establishing a data communication connection to the IMD using a patient device, the corresponding notification signal on the extracorporeal device is automatically cleared. The clearance can be initiated by a "clear notification" message which can be e.g. sent from the patient device to the extracorporeal device, or sent by the patient device to the IMD, and from the IMD to the extracorporeal device.

According to an example, the communication between IMD, extracorporeal device and patient device can be implemented as follows: First, the IMD and extracorporeal device can be configured to communicate by means of intra-body communication. Second, the IMD and patient device can be configured to communicate by means of an inductive coilbased communication. Third, the patient device and extracorporeal device (if they are able to communicate directly) can be configured to communicate via Bluetooth, Medical Implant Communication Service (MICS) band, or otherwise. The third part would require that both the patient device and the extracorporeal device to comprise a separate transceiver unit specifically for Bluetooth/MICS, in addition to the other transceiver units used to communicate with the IMD. In the context of the patient device, supporting this third communication path would require three separate transceiver units: an inductive coil transceiver unit, a CDMA/GSM/etc. transceiver unit for communication with the remote services infrastructure, and a Bluetooth/MICS transceiver unit for communication with the extracorporeal device.

In case a data communication connection to the IMD is established, the patient device enables sending the data collected by the IMD to a remote services infrastructure. Moreover, an access to the IMD can be provided to a user by means of the patient device or by means of a remote services infrastructure via the patient device. Such engagement provisions the patient's healthcare support team with access to collected data of the IMD, IMD device parameters, and patient status information.

Further, sending the data collected by the IMD to the remote services infrastructure comprises providing specific information related to the detected condition, e.g., AF snapshots, recognized by the IMD. Alternatively or additionally, the data comprises information/data for a follow-up examination, e.g. baseline follow-up data in support of a (nominally prescribed) device/patient monitoring regimen.

In the context of reporting of the IMD-observed condition alone, different icons or sounds may be used for distinct detected conditions. In cases where a data communication link is established, the user would be expected to place a wand-enabled patient device onto the patient's body (with or without intervening clothing) to initiate inductive communication with the deep IMD. Likely stemming from a user button press (or similar) following wandapplication, the patient device would exchange information with the IMD to perform interrogation routines by first locally storing the collected interrogation data, and then relaying the collected information to a remote services infrastructure. The patient device itself and/or in coordination with the extracorporeal device and remote monitoring services may then further serve to inform the patient of success/pending/failure/etc. conditions associated with attempts to deliver the collected information to the remote managed care infrastructure. In cases where the administration of medications might be recommended on the basis of IMD recognized conditions, a user acknowledgement of compliance would likely be supported by the extracorporeal device where user button presses (or otherwise) could silence the messaging. Such support would only be enabled in the system subject to the direction of the patient's attending clinician via program settings written to the IMD at the last follow-up. Furthermore, any medication based recommendations may be likely to be linked heavily with recommendations that the patient contact their doctor in cases where they do notify the patient via the extracorporeal device.

The termination signal used to halt the IMD's beaconing as previously discussed may be sent by the transceiver unit of the extracorporeal device or the wand-enabled patient device (the latter specific to notification-based cases that involve IMD interrogation) and received by the IMD, (i.e. its communication unit) in the former case via intra-body communication and in the latter via inductive communication. Termination signals may be preferably relayed after the patient has confirmed the receipt of the notification signal by interactions with the extracorporeal devices; once the extracorporeal device has successfully received the notice from the IMD; or subject to the execution of successful interrogation processes by the patient device. This silencing of the IMD's beaconing aids in its ability to avoid unnecessary power consumption and may nominally return it to a baseline monitoring condition where it once again can search for new, emergent patient/device conditions. In cases where an initially-recognized IMD/patient trigger persists, the system design would be formatted to support the capacity to turn off the IMD's monitoring and beaconing for such a condition - as repeatedly informing the clinical care team of a known condition would only shorten device service without offering added therapeutic benefit. As previously noted, the beaconing itself could furthermore be throttled using a time-out process where if it has not received a "stop" notification from another device it would automatically stop reporting the condition and presumably return to a monitoring status where it sought newly-emergent IMD/patient conditions. Again, such an approach would avoid unwanted power consumption and directly address cases where the extracorporeal device was not charged, not being worn, or had been damaged or failed in some manner.

According to a further example of the proposed method, the notification signal provided by the extracorporeal device comprises information from a remote service infrastructure. The information is either directly sent from the remote service infrastructure to the extracorporeal device, or from the remote service infrastructure via the patient device to the extracorporeal device. In the latter case the patient device serves as repeater for the remote service infrastructure.

Said information sent from the remote service infrastructure may comprise for example a notification for the patient of a requested behavior based on the guidance or prescription of the patient's clinical care provider. In such an example, the extracorporeal device would notify the patient regarding
1.) potential non-compliance with a remote follow-up regimen (reminding them to collect added data using their patient device for relay to the remote services infrastructure);
2.) potential non-compliance with a co-prescribed medication regimen (encouraging them to take their blood thinner, for example); and/or
3.) their need to visit a healthcare practitioner at some outlined regularity.

At large, the above outlined invention facilitates a means to ensure that in-office follow-up needs be no more frequent for leadless IMD systems than for traditional leaded implant devices. Such support is enabled by providing caretakers with added confidence in therapy support management between follow-up by enabling critical changes in device/patient interactions to be made known when they occur. In addition, it offers patient peace of mind that their miniaturized IMD provides critical clinical support capabilities that are well aligned with offerings from larger footprint, more power-hungry counterparts by provisioning remote monitoring with the smallest envisionable impact on IMD service time. The invention additionally extends cardiac rhythm management (CRM) care to enable a more holistic approach to patient support that marries device-based therapies with their affiliate needs for administering coordinated medication regimens.

Features described in association with the described method are analogously applicable to the described medical device system and method for operation of this system, and vice versa.

The present invention will now be described in further detail with reference to the accompanying schematic drawings, wherein
- Fig. 1A: shows an exemplary medical system with an active implantable medical device (IMD) implanted within a patient's body, more specific within a patient's heart,
- Fig. 1B: shows the IMD and an extracorporeal device in more detail,
- Fig. 2: depicts a flow chart of a method for operation of such medical system, and
- Fig. 3: depicts a flow chart of another method method for operation of such medical system.

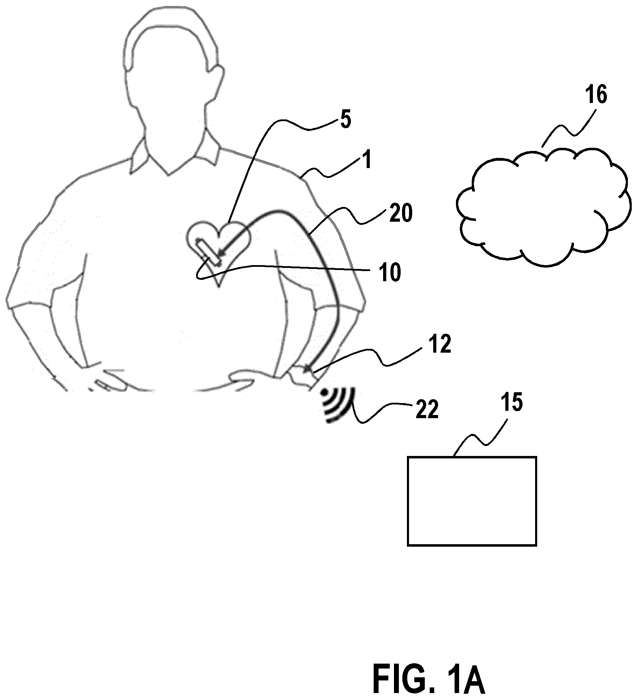

Fig. 1A shows an exemplary medical system for a patient 1 comprising an active implantable medical device (IMD) in the form of an implantable leadless pacemaker (ILP) 10 implanted within the heart 5 of the patient 1. Further, the patient wears an extracorporeal device 12 realized as a smart watch or wrist band at his/her wrist and is attached to the skin in the wrist region. Further, the system comprises another patient device 15 being equipped with a processing and data storage capability, a coilbased inductive transceiver unit for communication with the IMD 10 and CMDA, VOIP, or otherwise enabled transceiver unit for connecting to a remote services infrastructure (16) - said infrastructure serving as a data access portal for the patient's healthcare provider.

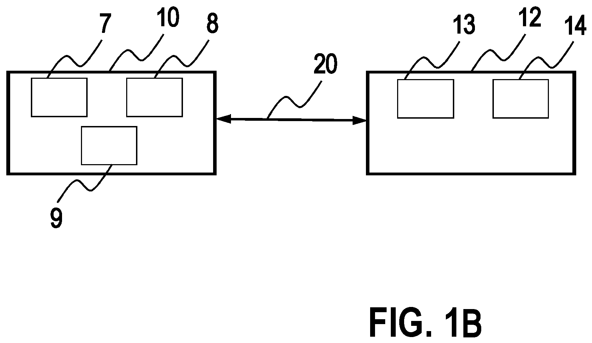

Fig. 1B shows the IMD 10 and the extracorporeal device 12 in more detail. The IMD 10 comprises a measurement unit 7, a processor 8 and a communication unit 9. The extracorporeal device 12 comprises a transceiver unit 13 and a notification unit 14 which are electrically connected.

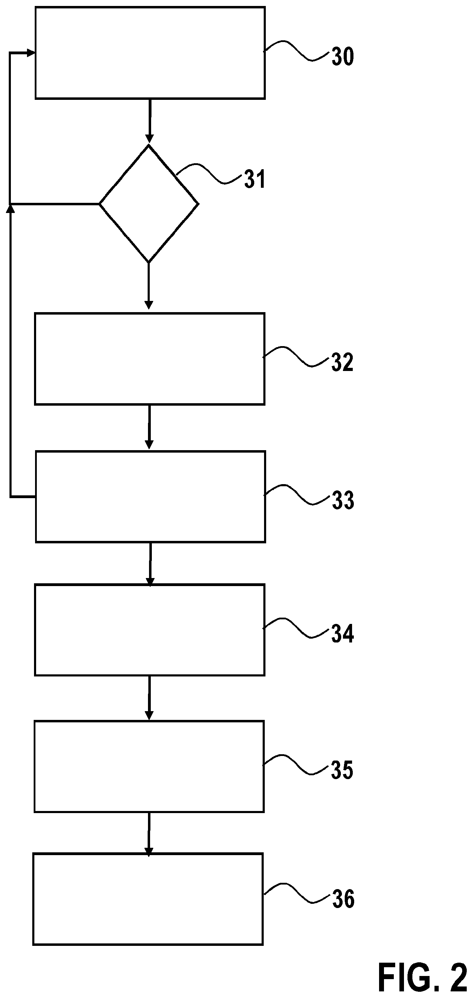

The steps of an exemplary method are depicted in Fig. 2 to which the following explanation of the method refers.

The measurement unit 7 of the IMD 10 determines continuously at least one bodily parameter of the patient's body, for example electric signals of the heart 5, e.g. an electrocardiogram, and sends the determined data of the bodily parameter to the processor 8 of the IMD 10 (see step 30) which is connected to the measurement unit 7. The processor 8 then, in step 31, analyses the received data of the bodily parameter and determines whether the data correspond to a predefined patient/device condition, for example an atrial fibrillation (AF). The at least one predefined condition and their criteria (for example threshold values) are stored in the storage unit of the IMD 10 in order to compare the received data of the at least one bodily parameter with them. In case detection of one of the predefined conditions is confirmed (step 32), the method continues with step 33 and the processor 8 activates the communication unit 9 of the IMD 10 and the communication unit 9 sends repeatedly, for example each 1/2 hour for the next 24 hours, a predefined intra-body communication signal through the body of the patient (depicted by arrow 20 of Fig. 1A) to the extracorporeal device 12 which is attached to the skin of the patient 1. In case no predefined condition is detected, the method continues with step 30.

The intra-body communication signal 20 may be realized as a short encoded beacon travelling through the body of the patient as explained above, for example an acoustic signal, an electrical measure or an impedance-modulated signal. In step 34 the transceiver unit 13 of the extracorporeal device 12 receives the intra-body communication signal 20 and the extracorporeal device provides a notification signal using its notification unit 14, i.e. it displays, for example, a respective icon and provides a beep showing the patient 1 that an atrial fibrillation condition has occurred and that the data associated with the detected atrial fibrillation needs to be collected by the patient device 15 for subsequent relay to the remote services infrastructure. The notification signal is depicted in Fig. 1A by the wave-symbol 22. As soon as, in step 35, the patient recognizes the notification signal 22 and confirms its receipt, e.g. by pressing a respective button of the extracorporeal device 12, the transceiver unit 13 sends a predefined intra-body communication signal 20 comprising a termination information to the communication unit 9 of the IMD 10. Alternatively or in combination, the extracorporeal device 12 sends a termination signal to IMD 10 via intra-body communication upon receiving the notification signal 22. Alternatively or in combination, if the intra-body communication signal 20 received by the transceiver unit 13 of the extracorporeal device 12 in step 34 requires the interrogation of IMD 10 via patient device 15, the extracorporeal device 12 displays said request to the patient 1. Patient 1 needs to perform the steps for said interrogation first before the receipt of notification signal 22 can be confirmed and before the termination information is sent to the communication unit 9 of the IMD 10. After successful interrogation, the patient device 15 may automatically inform the IMD 10 (directly or via extracorporeal device 12) that the intra-body communication signal 20 may be terminated. Alternatively or in combination, all notifications on the extracorporeal device 12 associated with the notification signal 22 are cleared upon successful interrogation of IMD 10. In the next step 36 the communication unit 9 receives the termination information and subsequently terminates sending the intra-body communication signal 20 by which the condition (e.g., "AF") is communicated to the extracorporeal device 12 reducing the power demands on the primary cell battery within the IMD 10.

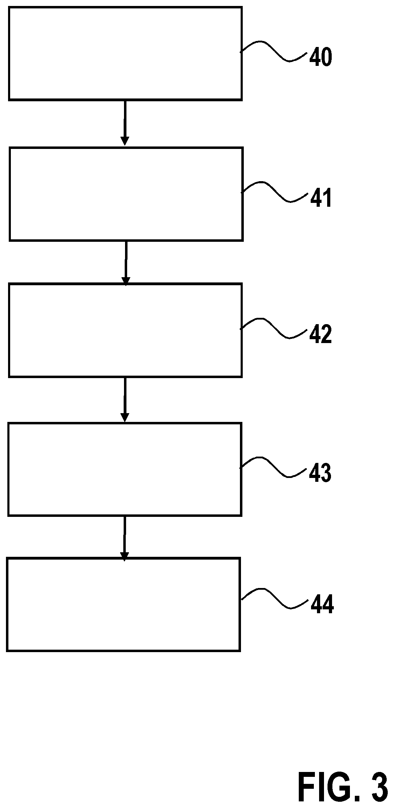

The steps of an exemplary method are depicted in Fig. 3 to which the following explanation of the method refers.

Notifications to the patient 1 can be transmitted from the remote services infrastructure 16 via patient device 15 to the extracorporeal device 12. In step 40, a request for such a notification is recognized in the remote services infrastructure 16. This can be initiated by a user, e.g. a physician, or automatically triggered by a periodical request (e.g. in the framework of a regular notification for a remote follow-up examination). In step 41, said request data is sent by from the remote services infrastructure 16 to the patient device 15. In step 42, patient device 15 forwards the request to extracorporeal device 12 where it is displayed as notification signal 22 to the patient. In step 43, the patient recognizes the notification signal 22 and confirms its receipt, e.g. by pressing a respective button of the extracorporeal device 12. Upon said confirmation, extracorporeal device 12 stops displaying notification signal 22 in step 44. Alternatively or in combination, after said confirmation a message is sent back via the patient device 15 to the remote services infrastructure 16 to inform the latter that the patient had acknowledged the request. Alternatively, if the notification signal 22 received the extracorporeal device 12 in step 43 requires the interrogation of IMD 10 via patient device 15, the extracorporeal device 12 displays said request to the patient 1. Patient 1 needs to perform the steps for said interrogation first before the receipt of notification signal 22 can be confirmed and before the notification signal 22 is stopped from being displayed by the extracorporeal device 12. After successful interrogation, the patient device 15 may automatically inform the extracorporeal device 12 (directly or via IMD 10) that displaying the notification signal 22 can be stopped. The patient device 15 may furthermore transport the collected information to the remote services center.

The above disclosure outlines a means for leveraging short, encoded messages from a deeply-implanted IMD 10 to inform the user of IMD/patient status through notifications emergent from an extracorporeal device 12 and request further patient interactions. This relaying of short, encoded messages (i.e. beacons) informs the recipient extracorporeal device 12 that the implanted IMD 10 has detected a patient or device condition that merits further survey or suggested needs for the administration of more proactive patient care measures. Additionally, it offers a means for tracking the system and/or patient with prescriptive measures outlined by the patient's clinical care team to either remind the patient to administer medications or that they should interact with their separate patient device to collect and relay data to remote, monitored resources. Within this framework, the extracorporeal device 12 directly informs the patient of such needs as part of its capacity for communicating with
1.) a remote services infrastructure (either directly or indirectly via relay through a separate patient device) and potentially also
2.) the IMD 10.

The data relay approaches that support the notification capabilities described herein (whether beaconing from the IMD 10 to the extracorporeal device 12 or messaging from the remote services infrastructure - either directly or indirectly, by way of the patient device 15 - to the extracorporeal device 12) generally contain minimal information, but passes sufficient content to the extracorporeal device 12 to inform the user that the active IMD 10 has witnessed a condition that merits further scrutiny or is reflective of indicators (potentially asymptomatic in nature) requiring patient actions to align with a co-prescribed medication regimen or, furthermore, pairs with a scheduled time point that merits "poking" the patient to remotely enhance care compliance.

The extracorporeal device 12 would, in a certain examples, present either iconography or legible messaging to the patient/wearer that
1.) encourages the application of the wand-based patient device 15, to inductively communicate further with the IMD 10 (in cases where added data could assist with clinical oversight) and/or
2.) calls attention to the patient regarding potential non-compliance with co-prescribed medication regimens (in cases where the implant alone recognizes key diagnostic signals or a time point prescribed by scheduling relayed from the remote monitoring infrastructure 16 has been reached), and/or
3.) comprises information/data for a follow-up examination, e.g. baseline follow-up data in support of a (nominally prescribed) device/patient monitoring regimen.

In case 2.), there is a data transmission first to the patient device 15 from the remote service center and a second data transmission form the patient device 15 to the extracorporeal device 12, for example in order to notify the patient of an activity based on assistance of a healthcare practitioner.

In the case where added data collection is driven by the implanted IMD 10 based upon algorithmic observations of key patient conditions, following the placement of the wand-enabled patient device 15 on the patient's body (over or near the IMD 10), the patient device 15 would perform an implant interrogation procedures to pull statistics, program/device settings, and potentially other log data from the IMD 10. This collected device information would, be re-transmitted to the remote service center by the patient device 15 to enable remote monitoring support. For cases where the extracorporeal device 12 indicated possible non-compliance with co-prescribed drug regimens, the patient would, confirm, through the device 12, that they had, in turn, responded by taking their medications or indicate that the reminder was unwarranted (potentially training the indicator algorithm as a result, whether it resides within the device or the remote services infrastructure). For cases where the extracorporeal device 12 indicates non-compliance to a prescription from the remote monitoring infrastructure 16 regarding the routine of follow-up examinations, the patient would confirm the message through device 12, wherein the message would reappear after a predetermined time period, relayed by the remote monitoring infrastructure 16 if the patient has still not undergone a follow-up examination.

## Claims

1. A medical system comprising an active implantable medical device (IMD, 10) and an extracorporeal device (12) attached to a patient's skin,
wherein the IMD (10) comprises
- a measurement unit (7) adapted to determining data regarding at least one bodily parameter of the patient (1) when the IMD (10) is implanted within a body of the patient (1),
- a processor (8) adapted to process data regarding the at least one bodily parameter and to detect at least one condition associated with a plurality of predefined conditions based on the data,
- a communication unit (9) adapted to periodically send an intra-body communication signal (20) representing the at least one detected condition to the extracorporeal device (12), wherein the extracorporeal device (12) comprises
- a transceiver unit (13) adapted to receive the intra-body communication signal (20),
- a notification unit (14) adapted to provide at least one predefined notification signal (22) to the patient (1) to motivate the patient (1) to perform a predefined action based on the received intra-body communication signal (20),
wherein the transceiver unit of the extracorporeal device (12) is further adapted to send a termination signal to the communication unit of the IMD (10), and
wherein the communication unit of the IMD (10) is further adapted to receive the termination signal and to subsequently terminate sending the intra-body communication signal (20) which was initiated by the previously at least one detected condition,
wherein the transceiver unit (13) of the extracorporeal device (12) is configured to send the termination signal to the communication unit (9) of the IMD (10) when the notification signal (22) is acknowledged by the patient (1).

2. The medical system according to claim 1, wherein the intra-body communication signal (20) is an acoustic signal, an electrical signal and/or an impedance-based signal.

3. The medical system of any of the preceding claims, wherein the notification signal (22) comprises a visual signal, haptic/tactile signal, and/or an acoustic signal.

4. The medical system of any of the preceding claims, wherein the extracorporeal device (12) is a smart watch, a wrist band, a mobile phone worn in direct physical contact with the patient's skin and/or a patch device.

5. The medical system of any of the preceding claims, wherein the notification signal (22) comprises
- a reporting to an user of the condition observed by the IMD (10),
- an invitation of the patient (1) to establish a data communication connection to the IMD (10) using a patient device (15) and/or
- an invitation of the patient (1) to take a medication and/or
- an invitation of the patient (1) to visit a healthcare professional.

6. The medical system of claim 5, wherein in case a data communication connection to the IMD (10) is established,
- the patient device (15) is configured to send the data collected by the IMD (10) to a remote services infrastructure, and/or
- the patient device (15) is configured to provide access to the IMD to a user by means of the patient device (15) or by means of the remote services infrastructure via the patient device (15).

7. The medical system of claim 6, wherein the data collected by the IMD (10) comprises
- specific information related to the detected condition recognized by the IMD (10) and/or
- data collected by or associated with the IMD for a follow-up examination.

8. The medical system of any of the preceding claims, wherein the notification signal (22) provided by the extracorporeal device (12) comprises information from a remote service infrastructure, wherein the information is sent
- from the remote service infrastructure to the extracorporeal device (12), or
- from the remote service infrastructure via the patient device (15) to the extracorporeal device (12).

9. A method for operation of a medical system comprising an active implantable medical device (IMD, 10) and an extracorporeal device (12) attached to a patient's skin, comprising the following steps:
- determining data regarding at least one bodily parameter of the patient (1) by a measurement unit (7) of the IMD (10) when the IMD (10) is implanted within a body of the patient (1),
- processing the data regarding the at least one bodily parameter and detecting at least one condition of a plurality of predefined condition based on the data by a processor (8) of the IMD (10),
- periodically sending an intra-body communication signal (20) representing the at least one detected condition by a communication unit (9) of the IMD (10) to the extracorporeal device (12),
- receiving the intra-body communication signal (20) by a transceiver unit (13) of the extracorporeal device (12),
- providing at least one predefined notification signal (22) by the extracorporeal device (12) to the patient (1) to motivate the patient (1) to perform a predefined action based on the received intra-body communication signal (20),
- sending a termination signal by the transceiver unit of the extracorporeal device (12) to the communication unit of the IMD (10), wherein the termination signal is sent by the transceiver unit (13) of the extracorporeal device (12) to the communication unit (9) of the IMD (10) when the notification signal (22) is acknowledged by the patient (1), and
- receiving the termination signal by the communication unit of the IMD (10) and subsequently terminating sending the intra-body communication signal (20) which was initiated by the previously at least one detected condition.

10. The method according to claim 9, wherein the notification signal (22) comprises
- a reporting to an user of the condition observed by the IMD (10),
- an invitation for the patient (1) to establish a data communication connection to the IMD (10) using a patient device (15) and/or
- an invitation of the patient (1) to take a medication and/or
- an invitation of the patient (1) to visit a healthcare professional

11. The method of claim 10, wherein in case a data communication connection to the IMD (10) is established,
- the patient device (15) enables sending the data collected by the IMD to a remote services infrastructure, and/or
- access to the IMD is provided to a user by means of the patient device (15) or by means of a remote services infrastructure via the patient device (15).

12. The method of claim 11, wherein sending the data collected by the IMD (10) to the remote services infrastructure comprises providing either
- specific information related to the detected condition recognized by the IMD (10) and/or
- information/data for a follow-up examination.

13. The method of any of the claims 9 to 12, wherein the notification signal (22) provided by the extracorporeal device (12) comprises information from a remote service infrastructure, wherein the information is sent
- from the remote service infrastructure to the extracorporeal device (12), or
- from the remote service infrastructure via the patient device (15) to the extracorporeal device (12).

## Patentansprüche

1. Medizinisches System mit einem aktiven implantierbaren medizinischen Gerät (IMD, 10) und einer extrakorporalen Vorrichtung (12), die an der Haut eines Patienten angebracht ist,
wobei das IMD (10) umfasst
- eine Messeinheit (7), die eingerichtet ist, Daten bezüglich mindestens eines Körperparameters des Patienten (1) zu bestimmen, wenn das IMD (10) in einen Körper des Patienten (1) implantiert ist,
- einen Prozessor (8), der eingerichtet ist, Daten bezüglich des mindestens einen Körperparameters zu verarbeiten und auf der Grundlage der Daten mindestens einen Zustand zu erkennen, der mit einer Vielzahl von vordefinierten Zuständen assoziiert ist,
- eine Kommunikationseinheit (9), die eingerichtet ist, periodisch ein intrakörperliches Kommunikationssignal (20), das den mindestens einen erkannten Zustand repräsentiert, an die extrakorporale Vorrichtung (12) zu senden, wobei die extrakorporale Vorrichtung (12) umfasst
- eine Sende-Empfangs-Einheit (13), die eingerichtet ist, das intra-körperliche Kommunikationssignal (20) zu empfangen,
- eine Benachrichtigungseinheit (14), die eingerichtet ist, dem Patienten (1) mindestens ein vordefiniertes Benachrichtigungssignal (22) bereitzustellen, um den Patienten (1) zu motivieren, eine vordefinierte Aktion auf der Grundlage des empfangenen intra-körperlichen Kommunikationssignals (20) durchzuführen,
wobei die Sende-Empfangs-Einheit der extrakorporalen Vorrichtung (12) ferner dazu eingerichtet ist, ein Beendigungssignal an die Kommunikationseinheit des IMD (10) zu senden, und
wobei die Kommunikationseinheit des IMDs (10) ferner dafür eingerichtet ist, das Beendigungssignal zu empfangen und anschließend das Senden des intra-körperlichen Kommunikationssignals (20) zu beenden, das durch den zuvor mindestens einen erkannten Zustand initiiert wurde,
wobei die Sende-Empfangs-Einheit (13) der extrakorporalen Vorrichtung (12) so konfiguriert ist, dass sie das Beendigungssignal an die Kommunikationseinheit (9) des IMDs (10) sendet, wenn das Benachrichtigungssignal (22) vom Patienten (1) bestätigt wird.

2. Das medizinische System nach Anspruch 1, wobei das intra-körperliche Kommunikationssignal (20) ein akustisches Signal, ein elektrisches Signal und/oder ein impedanzbasiertes Signal ist.

3. Das medizinische System nach einem der vorhergehenden Ansprüche, wobei das Benachrichtigungssignal (22) ein visuelles Signal, ein haptisches/taktiles Signal und/oder ein akustisches Signal umfasst.

4. Das medizinische System nach einem der vorhergehenden Ansprüche, wobei die extrakorporale Vorrichtung (12) eine Smartwatch, ein Armband, ein Mobiltelefon, das in direktem physischen Kontakt mit der Haut des Patienten getragen wird, und/oder ein Patch-Gerät ist.

5. Das medizinische System nach einem der vorhergehenden Ansprüche, wobei das Benachrichtigungssignal (22) umfasst
- eine Meldung des von dem IMD (10) beobachteten Zustands an einen Benutzer,
- eine Aufforderung des Patienten (1), unter Verwendung eines Patientengeräts (15) eine Datenkommunikationsverbindung zu dem IMD (10) herzustellen und/oder
- eine Aufforderung an den Patienten (1), ein Medikament einzunehmen und/oder
- eine Aufforderung des Patienten (1) zum Besuch einer medizinischen Fachkraft.

6. Das medizinische System nach Anspruch 5, bei dem eine Datenkommunikationsverbindung mit dem IMD (10) hergestellt wird,
- das Patientengerät (15) so konfiguriert ist, dass es die von dem IMD (10) erfassten Daten an eine Ferndienstinfrastruktur sendet, und/oder
- das Patientengerät (15) so konfiguriert ist, dass es einem Benutzer mit Hilfe des Patientengeräts (15) oder mit Hilfe der Ferndienstinfrastruktur über das Patientengerät (15) Zugang zu dem IMD gewährt.

7. Das medizinische System nach Anspruch 6, wobei die von dem IMD (10) erfassten Daten umfassen
- spezifische Informationen in Bezug auf den von dem IMD (10) erkannten Zustand und/oder
- Daten, die von dem IMD für eine Folgeuntersuchung erfasst wurden oder mit ihr assoziiert sind.

8. Das medizinische System nach einem der vorhergehenden Ansprüche, wobei das von der extrakorporalen Vorrichtung (12) bereitgestellte Benachrichtigungssignal (22) Informationen von einer Ferndienstinfrastruktur umfasst, wobei die Informationen gesendet werden
- von der Fernwartungsinfrastruktur an die extrakorporale Vorrichtung (12), oder
- von der Ferndienstinfrastruktur über das Patientengerät (15) an die extrakorporale Vorrichtung (12).

9. Verfahren zum Betrieb eines medizinischen Systems, das ein aktives implantierbares medizinisches Gerät (IMD, 10) und eine an der Haut eines Patienten angebrachte extrakorporale Vorrichtung (12) umfasst, mit den folgenden Schritten:
- Bestimmen von Daten bezüglich mindestens eines Körperparameters des Patienten (1) durch eine Messeinheit (7) des IMDs (10), wenn das IMD (10) in einen Körper des Patienten (1) implantiert ist,
- Verarbeiten der Daten bezüglich des mindestens einen Körperparameters und Erkennen mindestens eines Zustands aus einer Vielzahl von vordefinierten Zuständen auf der Grundlage der Daten durch einen Prozessor (8) des IMDs (10),
- periodisches Senden eines intra-körperlichen Kommunikationssignals (20), das den mindestens einen erkannten Zustand darstellt, durch eine Kommunikationseinheit (9) des IMDs (10) an die extrakorporale Vorrichtung (12),
- Empfang des intra-körperlichen Kommunikationssignals (20) durch eine Sende-Empfangs-Einheit (13) der extrakorporalen Vorrichtung (12),
- Bereitstellen mindestens eines vordefinierten Benachrichtigungssignals (22) durch die extrakorporale Vorrichtung (12) an den Patienten (1), um den Patienten (1) zu motivieren, eine vordefinierte Aktion auf der Grundlage des empfangenen intra-körperlichen Kommunikationssignals (20) durchzuführen,
- Senden eines Beendigungssignals durch die Sende-EmpfangsEinheit der extrakorporalen Vorrichtung (12) an die Kommunikationseinheit des IMDs (10), wobei das Beendigungssignal durch die Sende-Empfangs-Einheit (13) der extrakorporalen Vorrichtung (12) an die Kommunikationseinheit (9) des IMDs (10) gesendet wird, wenn das Benachrichtigungssignal (22) durch den Patienten (1) bestätigt wird, und
- Empfangen des Beendigungssignals durch die Kommunikationseinheit des IMDs (10) und anschließendes Beenden des Sendens des intra-körperlichen Kommunikationssignals (20), das durch den zuvor mindestens einen erkannten Zustand initiiert wurde.

10. Das Verfahren nach Anspruch 9, wobei das Benachrichtigungssignal (22) umfasst
- eine Meldung des von dem IMD (10) beobachteten Zustands an einen Benutzer,
- eine Aufforderung an den Patienten (1), unter Verwendung eines Patientengeräts (15) eine Datenkommunikationsverbindung zu dem IMD (10) herzustellen und/oder
- eine Aufforderung an den Patienten (1), ein Medikament einzunehmen und/oder
- eine Aufforderung des Patienten (1) zum Besuch einer medizinischen Fachkraft

11. Das Verfahren nach Anspruch 10, bei dem eine Datenkommunikationsverbindung mit dem IMD (10) hergestellt wird,
- das Patientengerät (15) das Senden der von dem IMD erfassten Daten an eine Ferndienstinfrastruktur ermöglicht, und/oder
- der Zugang zu dem IMD wird einem Benutzer mit Hilfe des Patientengeräts (15) oder mit Hilfe einer Ferndienstinfrastruktur über das Patientengerät (15) gewährt.

12. Das Verfahren nach Anspruch 11, wobei das Senden der von dem IMD (10) erfassten Daten an die Ferndienstinfrastruktur das Bereitstellen von entweder
- spezifischen Informationen in Bezug auf den von dem IMD (10) erkannten Zustand und/oder
- Informationen/Daten für eine Folgeuntersuchung umfasst.

13. Das Verfahren nach einem der Ansprüche 9 bis 12, wobei das von der extrakorporalen Vorrichtung (12) bereitgestellte Benachrichtigungssignal (22) Informationen von einer Ferndienstinfrastruktur umfasst, wobei die Informationen gesendet werden
- von der Ferndienstinfrastruktur an die extrakorporale Vorrichtung (12), oder
- von der Ferndienstinfrastruktur über das Patientengerät (15) an die extrakorporale Vorrichtung (12).

## Revendications

1. Système médical comprenant un dispositif médical implantable actif (IMD, 10) et un dispositif extracorporel (12) attaché à la peau d'un patient,
dans lequel l'IMD (10) comprend
- une unité de mesure (7) adaptée pour déterminer des données concernant au moins un paramètre corporel du patient (1) lorsque l'IMD (10) est implanté au sein d'un corps du patient (1),
- un processeur (8) adapté pour traiter des données concernant l'au moins un paramètre corporel et pour détecter au moins une condition associée à une pluralité de conditions prédéfinies en se basant sur les données,
- une unité de communication (9) adaptée pour envoyer périodiquement un signal de communication intracorporelle (20) représentant l'au moins une condition détectée au dispositif extracorporel (12), dans lequel le dispositif extracorporel (12) comprend
- une unité d'émetteur-récepteur (13) adaptée pour recevoir le signal de communication intracorporelle (20),
- une unité de notification (14) adaptée pour fournir au moins un signal de notification prédéfini (22) au patient (1) afin de motiver le patient (1) à réaliser une action prédéfinie en se basant sur le signal de communication intracorporelle (20) reçu,
dans lequel l'unité d'émetteur-récepteur du dispositif extracorporel (12) est en outre adaptée pour envoyer un signal d'arrêt à l'unité de communication de l'IMD (10), et dans lequel l'unité de communication de l'IMD (10) est en outre adaptée pour recevoir le signal d'arrêt et pour conséquemment arrêter l'envoi du signal de communication intracorporelle (20) qui a été initié par l'au moins une condition détectée précédemment,
dans lequel l'unité d'émetteur-récepteur (13) du dispositif extracorporel (12) est configurée pour envoyer le signal d'arrêt à l'unité de communication (9) de l'IMD (10) lorsque le signal de notification (22) est confirmé par le patient (1).

2. Système médical selon la revendication 1, dans lequel le signal de communication intracorporelle (20) est un signal acoustique, un signal électrique et/ou un signal basé sur impédance.

3. Système médical selon l'une quelconque des revendications précédentes, dans lequel le signal de notification (22) comprend un signal visuel, un signal optique/tactile et/ou un signal acoustique.

4. Système médical selon l'une quelconque des revendications précédentes, dans lequel le dispositif extracorporel (12) est une montre intelligente, un bracelet, un téléphone mobile porté en contact physique direct avec la peau du patient et/ou un dispositif de timbre.

5. Système médical selon l'une quelconque des revendications précédentes, dans lequel le signal de notification (22) comprend
- un rapport à un utilisateur de la condition observée par l'IMD (10),
- une invitation du patient (1) à établir une connexion de communication de données à l'IMD (10) en utilisant un dispositif patient (15) et/ou
- une invitation du patient (1) à prendre un médicament et/ou
- une invitation du patient (1) à rendre visite à un professionnel de santé.

6. Système médical selon la revendication 5, dans lequel dans le cas où une connexion de communication de données à l'IMD (10) est établie,
- le dispositif patient (15) est configuré pour envoyer les données recueillies par l'IMD (10) à une infrastructure de services à distance, et/ou
- le dispositif patient (15) est configuré pour fournir un accès à l'IMD à un utilisateur au moyen du dispositif patient (15) ou au moyen de l'infrastructure de services à distance par l'intermédiaire du dispositif patient (15).

7. Système médical selon la revendication 6, dans lequel les données recueillies par l'IMD (10) comprennent
- des informations spécifiques liées à la condition détectée reconnue par l'IMD (10) et/ou
- des données recueillies par ou associées à l'IMD pour un examen de suivi.

8. Système médical selon l'une quelconque des revendications précédentes, dans lequel le signal de notification (22) fourni par le dispositif extracorporel (12) comprend des informations d'une infrastructure de services à distance, dans lequel les informations sont envoyées
- depuis l'infrastructure de services à distance jusqu'au dispositif extracorporel (12), ou
- depuis l'infrastructure de services à distance par l'intermédiaire du dispositif patient (15) jusqu'au dispositif extracorporel (12).

9. Méthode d'utilisation d'un système médical comprenant un dispositif médical implantable actif (IMD, 10) et un dispositif extracorporel (12) attaché à la peau d'un patient, comprenant les étapes suivantes consistant à :
- faire déterminer des données concernant au moins un paramètre corporel du patient (1) par une unité de mesure (7) de l'IMD (10) lorsque l'IMD (10) est implanté au sein du corps du patient (1),
- faire traiter les données concernant l'au moins paramètre corporel et faire détecter au moins une condition d'une pluralité de conditions prédéfinies en se basant sur les données par un processeur (8) de l'IMD (10),
- faire envoyer périodiquement un signal de communication intracorporelle (20) représentant l'au moins une condition détectée par une unité de communication (9) de l'IMD (10) au dispositif extracorporel (12),
- faire recevoir le signal de communication intracorporelle (20) par une unité d'émetteur-récepteur (13) du dispositif extracorporel (12),
- faire fournir au moins un signal de notification prédéfini (22) par le dispositif extracorporel (12) au patient (1) pour motiver le patient (1) à réaliser une action prédéfinie en se basant sur le signal de communication intracorporelle (20) reçu,
- faire envoyer un signal d'arrêt par l'unité d'émetteur-récepteur du dispositif extracorporel (12) à l'unité de communication de l'IMD (10), dans lequel le signal d'arrêt est envoyé par l'unité d'émetteur-récepteur (13) du dispositif extracorporel (12) à l'unité de communication (9) de l'IMD (10) lorsque le signal de notification (22) est confirmé par le patient (1), et
- faire recevoir le signal d'arrêt par l'unité de communication de l'IMD (10) et conséquemment arrêter l'envoi du signal de communication intracorporelle (20) qui a été initié par l'au moins une condition détectée précédemment.

10. Procédé selon la revendication 9, dans lequel le signal de notification (22) comprend
- un rapport à un utilisateur de la condition observée par l'IMD (10),
- une invitation au patient (1) pour établir une connexion de communication de données à l'IMD (10) en utilisant un dispositif patient (15) et/ou
- une invitation du patient (1) à prendre un médicament et/ou
- une invitation du patient (1) à rendre visite à un professionnel de santé.

11. Méthode selon la revendication 10, dans laquelle dans le cas où une connexion de communication de données à l'IMD (10) est établie,
- le dispositif patient (15) permet l'envoi des données recueillies par l'IMD à une infrastructure de services à distance, et/ou
- l'accès à l'IMD est fourni à un utilisateur au moyen du dispositif patient (15) ou au moyen d'une infrastructure de services à distance par l'intermédiaire du dispositif patient (15).

12. Méthode selon la revendication 11, dans laquelle l'étape consistant à envoyer les données recueillies par l'IMD (10) à l'infrastructure de services à distance comprend une étape consistant à fournir
- des informations spécifiques liées à la condition détectée reconnue par l'IMD (10) et/ou
- des informations/données pour un examen de suivi.

13. Méthode selon l'une quelconque des revendications 9 à 12, dans laquelle le signal de notification (22) fourni par le dispositif extracorporel (12) comprend des informations d'une infrastructure de services à distance, dans lequel les informations sont envoyées
- depuis l'infrastructure de services à distance jusqu'au dispositif extracorporel (12), ou
- depuis l'infrastructure de services à distance par l'intermédiaire du dispositif patient (15) jusqu'au dispositif extracorporel (12).
